# EUROPEAN PATENT APPLICATION

(11) **EP 2 366 394 A1**
(43) Date of publication of application: **21.09.2011**
(21) Application number: 10156805.3
(22) Date of filing: 17.03.2010
(51) Int. Cl.: A61K 31/496, A61K 31/00, A61P 29/00, A61P 37/00, C12Q 1/37, G01N 33/50

(54) **Characterization and validation of inhibitors and ligands of dipeptidyl aminopeptidase IV (DP IV)**

(71) Applicant: IMTM GmbH, 39120 Magdeburg (DE)
(72) Inventor: Ansorge, Siegfried, 39291, Hohenwarthe (DE); Bank, Ute, 39418, Staßfurt (DE); Nordhoff, Karsten, 39118, Magdeburg (DE); Täger, Michael, 39326, Heinrichsberg (DE)
(74) Representative: Bauch-Koepe, Katharina Anna

(57) **Abstract**

The invention relates to a method for characterization and validation of inhibitors or ligands of DPIV, DP8 or DP9 by their interaction with specific binding sites *in silico* and *in vitro.* It describes the use of special binding properties at the binding site of the DPIV in the central pore of DPIV, which is distant from the catalytic domain by at least 10 Angström (1 nm) which is essential for the steric inhibition of this peptidase and related enzymes like DP8 and DP9 by DPIV ligands or steric inhibitors for the use of these compounds to suppress DNA synthesis of lymphocytes, dermal cells and other cells. This finding is also important in that this technique can be used to predict unwanted side effects of DPIV related drugs and exclude effects of DPIV inhibitors developed for the treatment of Diabetes mellitus type II on immune functions and vice versa.

## Description

The present invention relates to the characterization and validation of inhibitors and ligands of dipeptidyl peptidase IV (DPIV). More specifically, the invention relates to compounds suitable in the characterization and validation of DPIV inhibitors and ligands, and relates to methods wherein such compounds are utilized. Finally, the invention also relates to the medical aspects of such characterization and validation methods which may include the use of the above compounds and methods in connection with the treatment of Diabetes mellitus type II, immune diseases including autoimmune diseases, allergies, transplant rejections, chronic inflammatory skin diseases as well as chronic and acute neuronal diseases.

In an even broader context, the field of the present invention comprises novel insight into the structural details and structure-function relationship of membrane-bound cellular dipeptidyl peptidase IV (DPIV) as well as of other members of the DPIV gene family like DP8 and DP9 having an enzymatic effect analogous to dipeptidyl peptidase IV (DPIV) ("analogous enzymatic effect"). Furthermore, the invention relates to special binding properties at binding sites of the DPIV localized in the central pore of DPIV and defining a steric inhibition of this peptidase and related enzymes (DP8, DP9) by DPIV ligands or inhibitors. The invention also relates to the corresponding inhibiting effect on DNA synthesis and other cellular functions by such a steric or indirect inhibition.

Moreover, the present invention relates to a method of characterization and validation of these binding sites. In particular, the present invention relates to a method of identifying and characterizing novel ligands or inhibitors which are capable of suppressing DNA synthesis and proliferation of various cells and changing other functions of immune cells, dermal cells and other cells expressing the enzyme.

In addition, the present invention relates to a use of the afore-mentioned technique for optimizing inhibitors of membrane-bound cellular DPIV or related enzymes for a prophylaxis and a therapy of diseases showing an excessive immune response and having an inflammatory genesis, of neuronal diseases and of cerebral damage, of tumor diseases, of skin diseases and of Diabetes mellitus type II.

The present invention also relates to the characterization of one or more compound(s) of DPIV inhibitors and ligands according to the afore-mentioned description and relates to the use of said compounds for the manufacture of a medicament for a prophylaxis and therapy of diseases with exceeding immune response and having an inflammatory genesis. These diseases include, but are not restricted to, autoimmune diseases, arteriosclerosis, allergies, transplant rejections, neuronal disease, cerebral damages, skin diseases, tumor diseases as well as Diabetes mellitus type II.

The enzyme dipeptidyl peptidase IV (DPIV, CD26, EC 3.4.14.5) is a membrane-bound or soluble serine protease existing ubiquitously and catalyzing the hydrolysis of peptides from the N-terminal end thereof specifically after proline, and - to a lesser extent - after alanine, or - with restrictions - after further amino acids like serine, threonine, valine and glycine at the second position of the N-terminus by a cleavage of Xaa-Pro dipeptides.

Further, a number of other molecules were found to exhibit various degrees of structural homology to DPIV and having DPP-IV-like enzyme activity, particularly in being capable of cleaving similar sets of substrates. In accordance with Sedo, A. et al.: Dipeptidyl peptidase IV-like molecules: homologous proteins or homologous activities?, Biochimica et Biophysica Acta, 2001, 1550, 107 to 116, the following peptidases are members of the DP IV gene family (DASH-structure homologs) and show the same or a similar substrate specificity as dipeptidyl peptidase IV ("DPIV analogous substrate specificity" or "DPIV analogous enzymatic activity") and sharing various degrees of structural homology: inter alia Fibroblast activation protein α (Seprase), Dipeptidyl peptidase IV β, Dipeptidyl aminopeptidase like protein, N-Acetylated α-linked acidic dipeptidase, Quiescent cell proline dipeptidase; Dipeptidyl peptidase II (DP II), DPP6 (DP6), DPP8 (DP8) and DPP9 (DP9) [see also Chen, T. et al.: Dipeptidyl peptidase IV gene family, Kluwer Academic/Plenum Publishers, New York, 2003, 79 - 86; Chen, T. et al.: Adv. Exp. Med. Biol., 2003, 524, 79].

A substrate specificity analogous to DPIV was also found for attractin (mahagony protein) [J. S. Duke-Cohan et al.: J. Immunol., 1996, 156, 1714].

The above enzymes having the same or similar substrate specificity as dipeptidyl peptidase IV are also inhibited by DPIV inhibitors.

For DPIV, important biological functions were proved in different cell systems. This is true - *inter alia -* for
- the immune system [S. Ansorge et al.: Clin. Chim. Lab. Med., 2000, 47, 253 - 261; D. M. T. Yu: FEBS Journal 2010, 1 - 19; M. D. Gorrell: Cli. Science, 2005, 108, 1 - 16; T. Kähne et al.: Intern. J. Mol. Med., 1999, 4, 3; I. De Meester et al.: Advanc. Exp. Med. Biol., 2002, 524, 3; R. Yazbek et al.: Trends Pharmacol. Sciences, 2009, 30, 600 - 606; International Patent Application No. WO 01/89,569; International Patent Application No. WO 02/053,170; International Patent Application No. WO 2004/004,750];
- the neuronal system [International Patent Application No. WO 02/053,169];
- the fibroblasts [International Patent Application WO 2005/004,906];
- the keratinocytes [International Patent Application No. WO 02/053,170];
- the sebaceous gland cells/sebocytes [International Patent Application No. WO 03/077,935];
- as well as tumors [M. D. Gorrell: Cli. Science 108, 1-16, 2005].

The capability of DPIV, of specifically inactivating the incretory hormones Gastrin-inhibitory peptide (GIP) and Glucagon-like peptide (GLP) led to the development of a new therapeutic concept and drugs for treating glucose metabolic disorders [D. M. Evans: Drugs 5, 577, 2002; T. W. von Geldern et al.: Drug Dev. Res. 67, 627-642 (2006); S. Nordhoff et al.: Bioorg. Med. Chem. Letters 19, 6340-6345, 2009; A. I. Palalau et al.: Postgraduate Medicine 121, 70-100, 2009].

Apart from the use of DPIV inhibitors as pharmaceuticals in treatment of Diabetes mellitus type II, other inhibitors of DPIV could be shown to be effective in accepted animal models in treatment of an excessive immune response in chronic inflammatory processes and of cerebral damages, also *in vivo* [S. Ansorge et al.: Clin. Chim. Lab. Med. 47, 253 to 261, 2009; International Patent Application No. WO 01/89,569].

The inhibition of the dipeptidyl peptidase IV as well as the inhibition of enzymes having an analogous substrate specificity, results into a strong inhibition of the DNA synthesis of immune cells and, hence, into a strong inhibition of the cell proliferation as well as into a change of the cytokine production, particularly into an induction of the immunosuppressive cytokine TGF-β1 [International Patent Application No. WO 01/89,569; International Patent Application No. WO 02/053,170] as well as into an inhibition of the generation and release of inflammatory cytokines of the type TH1, e.g. interleukine-2 (IL-2) and TH2, e. g. interleukine-4 (IL-4) [International Patent Application No. WO 02/053,170], and TH17, e.g. II-17 [S. Ansorge et al.: Clin. Chim. Lab. Med. 47, 253 to 261, 2009]. In the neuronal system, a decrease or retardation of acute and chronic cerebral damage processes was proved by an inhibition of DPIV and Alanylaminopepidase N [International Patent Application No. WO 02/053,169]. Moreover, it was proved for fibroblasts [International Patent Application No. WO 2005/004,906], keratinocytes [International Patent Application No. WO 02/053,170] and sebocytes [International Patent Application No. WO 03/077,935] that inhibition of DPIV effects an inhibition of the cell growth and a change of the cytokine production [A. Thielitz et al.: J. Invest. Dermatol. 127, 1042-1051, 2007; A. Thielitz et al.: J. Invest. Dermatol. 128, 855-866, 2008].

It results from the above prior art that the dipeptidyl peptidase IV as well as enzymes having an DPIV-analogous enzymatic activity perform fundamental central biologic functions in different organs and cell systems, and that an inhibition of DPIV represents an effective therapeutic principle for the treatment of various diseases, particularly Diabetes mellitus type II, and chronic inflammatory diseases, like autoimmune diseases, allergies and graft rejections.

In the past, all inhibitors of DPIV and all inhibitors of enzymes having a DPIV-analogous enzymatic activity were characterized routinely by their capacity to inhibit the hydrolysis of low molecular artificial substrates of the basic structure of an Xaa-Pro-acyl amide, as for example of the formula Gly-Pro-acyl amide, like Gly-Pro-para-nitroanilide, i.e. by the inhibitors' interactions with the active sites of these serine peptidases, specifically with the active site of the serine peptidase DPIV (which is the site characterized by the residues Glu205 - Glu206 - Arg358 - Arg125 - Ser630) and the removal of the dipeptide and the amine.

Concerning the effect of DPIV inhibitors on the modulation of the immune response like proliferation of lymphocytes or cytokine production, we now surprisingly found that a number of potent DPIV inhibitors are not capable to inhibit lymphocyte proliferation significantly.

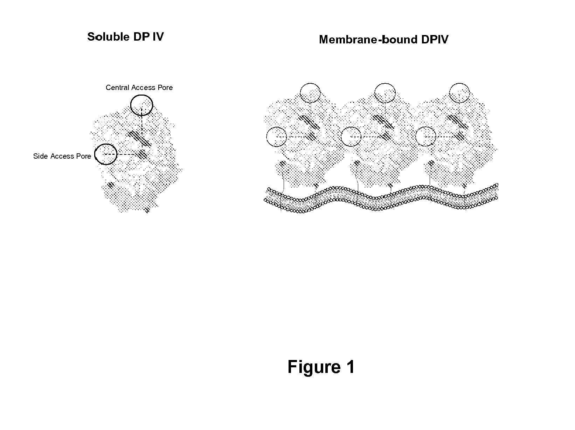

Studying these effects more in detail by using in silico docking approaches on the crystal structure model of DPIV [H.B. Rasmussen et al.: Nature Struct. Biol. Advanced online publication 2002], we surprisingly found that additional binding sites are involved in mediating the effects of DPIV inhibitors on immune response. These binding sites are located within the central pore of DPIV, which connects the environment of the enzyme to its above mentioned active site (Glu205 - Glu206 - Arg358 - Arg125 - Ser630) and which has its opening opposite to the membrane to which the membrane-bound cellular DPIV is attached (Figure 1).

More preferably, we found a strong correlation of binding of ligands and inhibitors to binding sites in the central pore, which binding sites are at least 10 Angström (1 nm) distant from Glu205 - Glu206 of the active site, and the suppression of DNA synthesis of immune cells like human T lymphocytes and mononuclear cells from peripheral blood, but also to the suppression of DNA synthesis of dermal cells like sebocytes, keratinocytes as well as fibroblasts and other DPIV expressing cells.

In an even more preferred manner, strongest effects on proliferation were found in cases where the ligands or inhibitors interact with DPIV amino acid residues His363, Glu361 and Glu408 of the binding sites, which amino acid residues are about 20 Angström (2 nm) distant from the residues Glu205 - Glu206 of the active site. Furthermore optimum binding was found for compounds with a logP value of approximately 3.5.

Inhibitors of DPIV and inhibitors of enzymes having a DPIV-analogous enzymatic effect which do not meet these criteria although they have a high inhibitory activity and a high selectivity for DPIV are not capable to suppress DNA synthesis significantly (Table III).

This finding is also important in that this technique enables a skilled person to predict unwanted side effects of drugs and exclude effects of DPIV inhibitors developed for the treatment of Diabetes mellitus type II on immune functions and vice versa.

One possible explanation for these discrepancies in the action of DPIV inhibitors on the degradation of GLP-1 (Diabetes) and the action on immune cells is the fact that, in case of DPIV-catalyzed GLP-1 processing, soluble DPIV is the main catalyst, whereas in case of DPIV mediated immunoregulation, the catalyst is membrane-bound cellular DPIV. Moreover, in case of the membrane-bound DPIV, it must be taken into consideration that DPIV molecules are embedded in a number of other proteins, and that under activation conditions membrane peptidases are clustering and forming so-called rafts (Figure 1).

Under these conditions the side access which is freely accessible in soluble DPIV might be blocked, and the major access is proposed to be the central pore opposite to the membrane (Figure 1).

This is a total different scenario in comparison to the incretin (GLP-1) inactivation by soluble DPIV in blood in that, in case of immunoregulation, peptide substrates will mainly enter the DPIV molecule on lymphocytes via the central pore. When peptide substrates are sterically inhibited from reaching the active site by DPIV inhibitors and ligands bound to the central pore binding sites, such peptide substrates will not reach the catalytically active binding site of membrane-bound cellular DPIV or of enzymes having a DPIV-analogous enzymatic activity.

Consequently, drug candidates on the basis of DPIV inhibitors have to be carefully characterized to exclude unwanted side effects, in case of antidiabetic drugs: immunomodulating effects, and in case of immunomodulating drugs: endocrine, glucose regulating effects.

Hence, the invention relates to a composition comprising (i) at least one member selected from dipeptidyl peptidase IV (DPIV) and an enzyme having DPIV-analogous enzymatic activity; and (ii) at least one steric inhibitor of dipeptidyl peptidase IV (DPIV) or of an enzyme having DPIV-analogous enzymatic activity, wherein said at least one steric inhibitor is bound to a binding site within the central pore of DPIV or of the enzyme having DPIV-analogous enzymatic activity.

Preferred embodiments of the invention are claimed in dependent claims 2 and 3.

The invention further relates to a method for an *in silico* or *in vitro* characterization of chemical compounds as steric inhibitors for dipeptidyl peptidase IV (DPIV) or for enzymes having DPIV-analogous enzymatic activity, which method comprises the steps of (i) selecting a compound for the characterization; (ii) bringing said selected compound into contact with dipeptidyl peptidase IV (DPIV) or with an enzyme having DPIV-analogous enzymatic activity; (iii) binding said compound to a binding site within the central pore, preferably to at least one of the amino acids His363 - Glu361 - Glu408, which is distant from the active site of DPIV (Glu205 - Glu206) or of the enzyme having DPIV-analogous emzymatic activity by at least 10 Angström (1 nm), preferably by 20 Angström (2 nm); and (iv) determining whether said compound binds to said binding site within the central pore of DPIV or of the enzyme having DPIV-analogous enzymatic activity, wherein the crystal structure of DPIV or of the enzyme having DPIV-analogous enzymatic activity is used as a target.

Preferred embodiments of the method of the invention are claimed in the dependent claims 6 and 7.

The invention further relates to the use of the method described in more detail below for a characterization of pharmacokinetic, pharmacodynamic, toxicologic and immunotoxicologic effects of inhibitors or ligands of DPIV or of an enzyme having DPIV-analogous enzymatic activity.

Moreover, the invention relates to the use of the method described in more detail below for a development or an optimization of inhibitors or ligands of DPIV or of an enzyme having DPIV-analogous enzymatic activity.

Further uses are claimed in the dependent claims 9 and 10.

Moreover, the invention relates to novel pharmaceutical preparations comprising at least one compound selected from the group consisting of the compounds shown in the Table of claim 11, optionally together with further pharmaceutically acceptable auxiliary substances and excipients.

The invention also relates to the use of the compounds selected from the group consisting of the compounds shown in the Table of claim 12 in the medical field.

Further, the invention relates to the use of the compounds selected from the group consisting of the compounds shown in the Table of claim 12 as steric inhibitors for DPIV or for enzymes having DPIV-analogous enzymatic activity *in silico* or *in vitro.*

Finally, the invention relates to the use of the compounds selected from the group of the compounds shown in the Table of claim 12 for manufacturing a medicament for the treatment of
- inflammatory diseases, preferably of autoimmune diseases, allergies, transplant rejection;
- chronic inflammatory skin diseases; or
- chronic or acute neuronal diseases.

The invention is in further detail explained by referring to the enclosed Figure 1, which shows the surface of the dimer of DPIV, based on the crystal structure of human DPIV (H. Rasmussen, loc. cit.) with the two access pores and the localization of the active site and the central pore binding site. Figure 1 also shows the comparison of soluble and membrane-bound DPIV. The figure is not intended to restrict the scope of the present invention.

The invention is now in detail described by referring to its preferred embodiments. It is, however, understood that the description of the invention by referring to preferred embodiments can, by no means, be construed as restricting the invention to those preferred embodiments.

In a first aspect, the invention relates to a composition which comprises (i) at least one member selected from dipeptidyl peptidase IV (DPIV) and an enzyme having DPIV-analogous enzymatic activity; and (ii) at least one steric inhibitor of dipeptidyl peptidase IV (DPIV) or of an enzyme having DPIV-analogous enzymatic activity, wherein said at least one steric inhibitor is bound to a binding site within the central pore of DPIV or of the enzyme having DPIV-analogous enzymatic activity.

The enzyme "dipeptidyl peptidase IV (DPIV)", which is also referred to as "CD 26" or "E.C. 3.4.14.5" as used in the present specification and in the claims, is a membrane-bound or soluble serine protease. It cleaves Xaa-Pro dipeptides from the N-terminus of peptides and proteins, and Xaa-Ala dipeptides are also cleaved to a lesser extent.

Dipeptidyl peptidase IV was believed to be a unique membrane-bound cellular protease cleaving Xaa-Pro dipeptides from the N-terminal end of peptides and proteins. There was further found a number of other enzyme molecules exhibiting various degrees of structural homology and DPIV-like enzyme activity, which enzyme molecules are capable of cleaving similar set of substrates.

In the present invention, dipeptidyl peptidase IV (DPIV) is preferably considered as the enzyme for which steric inhibitors are sought, and DPP8 and DPP9 are preferably considered as the enzymes having DPIV-analogous enzymatic activity.

In accordance with the invention, the composition comprises at least one member selected from DPIV and an enzyme having DPIV-analogous enzymatic activity. In preferred embodiments of the invention, the composition may comprise one enzyme, two enzymes, three enzymes or even more enzymes selected from the group consisting of DPIV and an enzyme having DPIV-analogous enzymatic activity. Further preferred in accordance with the invention are compositions which comprise one enzyme selected from the group consisting of DPIV and an enzyme having DPIV-analogous enzymatic activity. Even more preferred are compositions of the invention which comprise DPIV as the enzyme.

In accordance with the invention, the composition also comprises at least one steric inhibitor selected from steric inhibitors of dipeptidyl peptidase IV (DPIV) and of an enzyme having DPIV-analogous enzymatic activity. The composition may comprise one steric inhibitor of DPIV and/or one steric inhibitor of an enzyme having DPIV-analogous enzymatic activity or may contain several (for example two, three) steric inhibitors of DPIV and/or several (for example two, three) steric inhibitors of an enzyme having DPIV-analogous enzymatic activity. Compositions comprising one steric inhibitor of dipeptidyl peptidase IV (DPIV) and/or one steric inhibitor of an enzyme having DPIV-analogous enzymatic activity are preferred, and even more preferred are compositions of the invention which comprise one steric inhibitor of DPIV.

The term "inhibitor of DPIV", as used in the present specification and claims, is understood to mean such substances which are able to specifically inhibit the enzyme activity of DP IV by binding to the active site (Glu205 - Glu206 - Arg 358 - Arg125 - Ser630). The corresponding meaning is applicable to the term "inhibitor of an enzyme having DPIV-analogous enzymatic activity"; said term is understood to mean substances which are able to specifically inhibit the enzymatic activity of any of the other enzymes having a substrate specificity similar to the one exhibited for DPIV. These inhibitors may belong to different structural groups.

The common characteristic feature of these inhibitors is their affinity to the active site of the dipeptidyl peptidase IV (DP IV) and of enzymes having a similar substrate specificity. This molecular site of the dipeptidyl peptidase IV and other enzymes having a similar substrate specificity is characterized by the following amino acid residues: Ser630, Asp708, His740 (catalytic triad), Glu205, Glu206 and Tyr547. Further inhibitor-binding amino acid residues are Tyr666, Phe357 and Arg358. These molecular basic principles of the specific interaction between inhibitors of dipeptidyl peptidase IV and dipeptidyl peptidase IV or of enzymes having a similar substrate specificity ("DPIV-analogous enzymatic activity") are the basis for a generalization independent of the specific structure of the inhibitors concerning the action and the biological role of these inhibitors deriving from results of established inhibitors.

The term "ligand of DPIV", as used in the present specification and claims and synonymously used herein with the term "steric inhibitor", is understood to mean such substances which are able to indirectly, or sterically, inhibit the DPIV enzymatic activity, i. e. by preventing substances (e. g. substrate molecules) from accessing the catalytically active site of the enzyme DPIV, for example through one of the access pores, preferably through the central access pore. The corresponding meaning is applicable to the term "ligand/steric inhibitor of an enzyme having DPIV-analogous enzymatic activity"; said term is understood to mean substances which are able to specifically inhibit the enzymatic activity of any of the other enzymes having a substrate specificity similar to the one exhibited for DPIV by indirect, or steric, inhibition.

In accordance with the present invention, said at least one steric inhibitor is bound to a binding site within the central pore of DPIV or of the enzyme having DPIV-analogous enzymatic activity.

The term "comprise" as used in the present specification and claims, for example in claim 1, has the meaning that a composition of the invention may comprise (i) at least one member selected from dipeptidyl peptidase IV (DPIV) and an enzyme having DPIV-analogous enzymatic activity; and (ii) at least one steric inhibitor selected from steric inhibitors of dipeptidyl peptidase IV (DPIV) and of an enzyme having DPIV-analogous enzymatic activity, wherein said at least one steric inhibitor is bound to a binding site within the central pore of DPIV or of the enzyme having DPIV-analogous enzymatic activity, and that further components (more specifically defined below) may also be comprised by the composition.

The term "comprise" as used in the present specification and claims may, however, also include cases where the composition of the invention mainly consists of (i) at least one member selected from dipeptidyl peptidase IV (DPIV) and an enzyme having DPIV-analogous enzymatic activity; and (ii) at least one steric inhibitor selected from steric inhibitors of dipeptidyl peptidase IV (DPIV) and of an enzyme having DPIV-analogous enzymatic activity, wherein said at least one steric inhibitor is bound to a binding site within the central pore of DPIV or of the enzyme having DPIV-analogous enzymatic activity, together with any necessary component a skilled person may include into such a composition in order to achieve the object of the invention, or may even include cases where the composition of the invention exclusively consists of (i) at least one member selected from dipeptidyl peptidase IV (DPIV) and an enzyme having DPIV-analogous enzymatic activity; and (ii) at least one steric inhibitor selected from steric inhibitors of dipeptidyl peptidase IV (DPIV) and of an enzyme having DPIV-analogous enzymatic activity, wherein said at least one steric inhibitor is bound to a binding site within the central pore of DPIV or of the enzyme having DPIV-analogous enzymatic activity.

In accordance with the invention, the at least one steric inhibitor, preferably the one steric inhibitor, more preferably the one steric inhibitor of DPIV, is bound to a binding site or, more precisely, to binding sites within the central pore of DPIV or of the enzyme having DPIV-analogous enzymatic activity. Such binding sites are located within the central pore of DPIV, which connects the environment of the enzyme to its above active site (Glu205 - Glu206 - Arg358 - Arg 125 - Ser630) and which has its opening opposite to the membrane to which the membrane-bound cellular DPIV is attached (Figure 1).

In a preferred embodiment of the composition according to the invention, said at least one steric inhibitor and, even more preferred, said one steric inhibitor and, mostly preferred, said steric inhibitor of DPIV, is bound to a binding site within the central pore of DPIV or of the enzyme having DPIV-analogous enzymatic activity. Said binding site is distant from the active site of DPIV (Glu205 - Glu206) or of the enzyme having DPIV-analogous enzymatic activity by at least 10 Angström (1 nm). In an even more preferred embodiment of the composition of the invention, said binding site is distant from the active site of DPIV (Glu205 - Glu206) or of the enzyme having DPIV-analogous enzymatic activity preferably by 20 Angström (2 nm).

Such distances between the binding site of the steric inhibitor within the central pore of DPIV (or of an enzyme having DPIV-analogous enzymatic activity) and the active site of DPIV (Glu205 - Glu206) or for the enzyme having DPIV-analogous enzymatic activity may be determined from the crystal structure of the enzyme, for example (but without restriction) of dipeptidyl peptidase IV (DPIV) by determining the distance rₐₛ between the carbonyl carbon atom of Glu206 (which is one of the amino acid residues representing the active site of the enzyme) and the centre of mass of the ligand or steric inhibitor.

More preferred compositions of the invention are those, wherein said at least one steric inhibitor is bound to at least one of the amino acids His363 - Glu361
- Glu408 within the binding site of the central pore of DPIV. This embodiment is preferred due to the fact that a desired and good steric inhibition of DPIV can be achieved.

In the course of making the present invention, the inventors found that a number of known compounds are suitable as steric inhibitors for DPIV or for an enzyme having a DPIV-analogous enzymatic activity. However, there were also found novel compounds suitable as steric inhibitors which were found to bind to the central pore binding site of DPIV, preferably to at least one of the amino acids His363 - Glu361 - Glu408. The novel steric inhibitors of the invention comprised by the particularly preferred compositions are shown in the subsequent Table I:

**Table I**

| **Structure** | **IUPAC name** | **No.** |
|---|---|---|
| 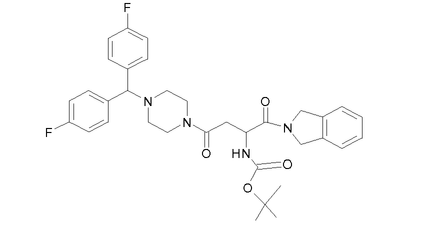 | 2-Oxy-methoxy-amino-1-(1,3-dihydro-2*H*-isoindol-2-yl)-4-(4-bis(4-fluorophenyl)methyl pipe-razine-1-yl*)butane-1,4-dione | 3 |
| 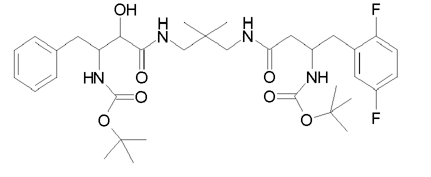 | 3-N-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 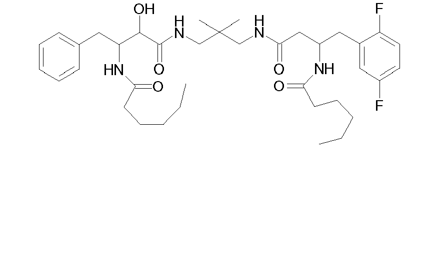 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 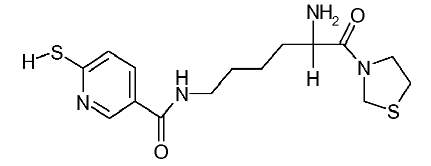 | *N*-[5-Amino-6-oxo-6-(1,3-th iazol id ine-3-yl)hexyl]-6-mercapto nicotinamide | 8 |
| 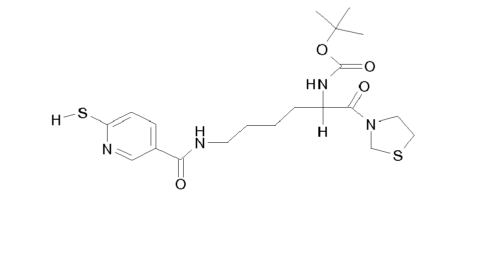 | 5-{[(6-Mercaptopyridine-3-yl)carbonyl]amino}-1-(1,3-thiazolidine-3-ylcarbonyl)pentyl carbamic acid (2,2 - dimethylethyl) ester | 9 |
| 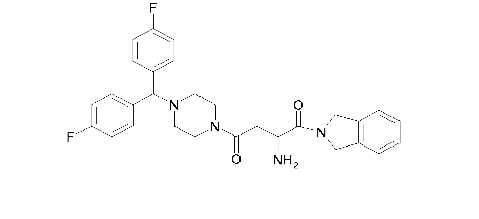 | 2-Amino-1-(1,3-dihydro-2*H*-isoindol-2-yl)-4-(4-bis(4-fluorophenyl)-methylpiperazine-1-yl) butane-1,4-dione | 13 |
| 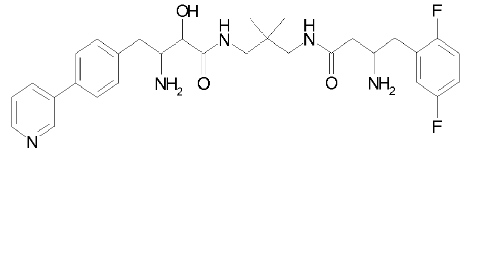 | 3-Amino-5-(4-(3-pyridinyl)-phenyl)-*N*-{3-[(3-amino-2-hydroxypropanoyl)-amino]-2,2-dimethyl propyl}-5-(2,5-difluorophenyl) pentane amide | 14 |
| 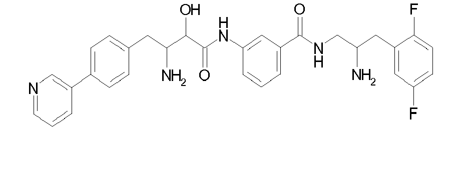 | 3-[(3-Amino-2-4-(4-(3-pyridinyl)- phenyl) hydroxybu-tanoyl)-amino]-*N*-(2-amino-5-(2,4-difluorophenyl) pentyl) ben-zamide | 15 |

All novel compounds shown in Table I are surprisingly suitable as steric inhibitors or ligands for DPIV or for an enzyme having DPIV-analogous enzymatic activity, preferably as steric inhibitors or ligands for DPIV and for DP8 and/or DP9. Particularly, these compounds are comprised by the most preferred compositions of the present invention, wherein at least one, more preferably one, steric inhibitor is bound to the DPIV HIS363 - Glu361 - Glu408 central pore binding site of DPIV. Details of such compositions mostly preferred and advantageously used in the present invention are shown in the experimental part of the description.

In accordance with another aspect of the invention, a method for an *in silico* or *in vitro* characterization of chemical compounds as steric inhibitors for dipeptidyl peptidase IV (DPIV) or for enzymes having DPIV-analogous enzymatic activity is claimed, which method comprises the steps of
(i) selecting a compound for the characterization;
(ii) bringing said selected compound into contact with dipeptidyl peptidase IV (DPIV) or with an enzyme having DPIV-analogous enzymatic activity;
(iii) binding said compound to a binding site within the central pore, preferably to at least one of the amino acids His363 - Glu361 - Glu408, which is distant from the active site of DPIV (Glu205 - Glu206) or of the enzyme having DPIV-analogous enzymatic activity by at least 10 Angström (1 nm), preferably by 20 Angström (2 nm);
(iv) determining whether said compound binds to said binding site within the central pore of DPIV or of the enzyme having DPIV-analogous enzymatic activity and restrict or even prevent the access to the active site;
wherein the crystal structure of DPIV or related models of enzymes having DPIV-analogous enzymatic activity is used as a target.

Due to their excellent and predictable properties as inhibitors of DPIV or of enzymes having DPIV-analogous enzymatic properties, steric inhibitors of DPIV or of enzymes having DPIV analogous enzymatic activity are sought for. Surprisingly, the method of the present invention is capable of characterizing chemical compounds with the aim to find compounds suitable for the above purpose. In accordance with the invention, it was surprisingly found that a number of well known inhibitors of DPIV are suitable as steric DPIV inhibitors. However, also novel compounds could be elucidated in accordance with the method of the invention which are suitable as excellent steric inhibitors of DPIV or of enzymes having DPIV-analogous enzymatic activity.

In the process of the invention, one of the steps is a selection of a compound which is to be subjected to the characterization process. The selection of such compounds can easily be made by a skilled person on the basis of the parameters mentioned below in the description and in the experimental part. Particularly, the structure of DPIV revealed in the form of a crystal structure was an excellent target which could be used for this step.

In another step of the method of the invention, said selected compound is brought into contact with dipeptidyl peptidase IV (DPIV) or with an enzyme having DPIV-analogous enzymatic activity. In a particularly preferred embodiment of the invention, such a contact is established on the basis of calculated structures of the enzyme included, on the one hand, and on the basis of the calculated structure of the steric inhibitor, on the other hand. Such a process is generally, and also in the frame of the present invention, named "an *in silico* process", i. e. a process conducted by computational methods or even computerized methods.

In another preferred embodiment of the method of the invention, a compound selected as a compound for the characterization may be brought in contact to dipeptidyl peptidase IV (DPIV) or to an enzyme having DPIV-analogous enzymatic activity *in vitro.* This may be done by methods known to a skilled person per se, i. e. in well-known *in-vitro* test or assay methods as those described below in the experimental part of the description.

In another step of the method according to the invention, the selected compound brought into contact to DPIV or to an enzyme having DPIV-analogous enzymatic activity is bound to a binding site within the central pore, preferably to at least one of the amino acids His363 - Glu361 - Glu408, which is distant from the active site of DPIV (Glu205 - Glu206) or of the enzyme having DPIV-analogous enzymatic activity by at least 10 Angström (1 nm). It was found surprisingly in the method of the present invention that a number of selected compounds, for example the compounds shown in the subsequent Table II, can be bound to said central pore binding site with a binding constant K_{cp} having a small value (representing a stable bond).

Also in this case, preferred embodiments of the method represent that the step is performed in *in silico* methods, resulting in good figures for the binding of the compound to DPIV or to an enzyme having a DPIV-analogous enzymatic activity. Other preferred embodiments show that *in-vitro* methods allow a characterization of chemical compounds as steric inhibitors or ligands of DPIV and of enzymes having DPIV-analogous enzymatic activity as well.

In a further preferred method of the present invention, said compound selected for the characterization and brought into contact with the enzyme, preferably with DPIV, is found to be bound to a binding site within the central pore which is distant from the active site of DPIV (Glu205 - Glu 206) preferably by 20 Angström (2 nm).

In a final step of the method of the invention, it is determined - again preferably *in silico* or *in vitro -* whether said compound binds to said binding site within the central pore of DPIV or of the enzyme having DPIV-analogous enzymatic activity.

In a preferred embodiment of the invention, the crystal structure of DPIV or of the enzyme having DPIV-analogous enzymatic activity is used as a target.

In accordance with a preferred embodiment, the method according to the invention is calibrated by using the following compounds of Table II as inhibitors or ligands for dipeptidyl peptidase IV (DPIV) or for enzymes having DPIV-analogous enzymatic activity:

**Table II**

| **Structure** | **IUPAC name** | **No.** |
|---|---|---|
| 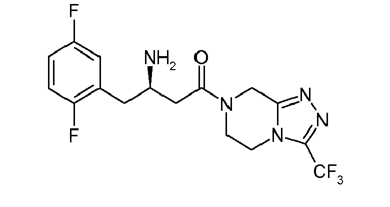 | (1*R*)-1-(2,5-Difluorobenzyl)-3-oxo-3-[3-(trifl uoromethyl)-5,6-dihydro[1,2,4]triazolo[4,3-a]pyrazine-7(8*H*)-yl]propylamine | 1 |
| 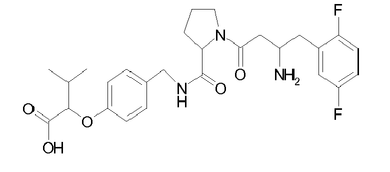 | 2-[({[1-(3-Amino, 2-(2, 5)-difluorophenylbutanoyl)pyrrolidin-2-yl]carbonyl}amino)-p-phenoxy]-3-methyl butanoic acid | 2 |
| 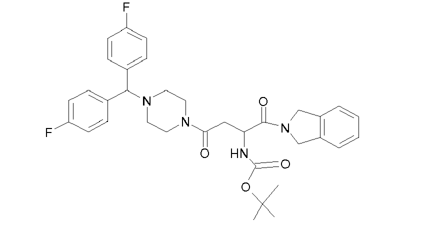 | 2-Oxy-methoxy-amino-1-(1,3-dihydro-2*H*-isoindol-2-yl)-4-(4-bis(4-fluorophenyl)methyl piperazine-1-yl)butane-1,4-dione | 3 |
| 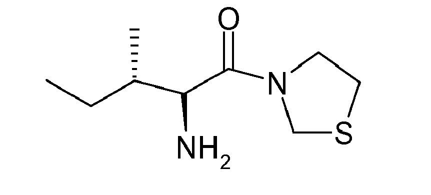 | (1*S*,2*S*)-2-Methyl-1-(1,3-thiazolidine-3-ylcarbonyl) butyl amine | 4 |
| 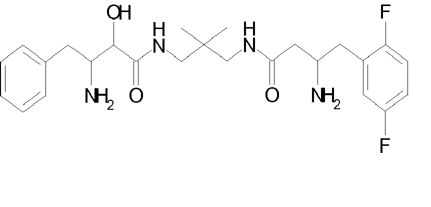 | 3-Amino-5-phenyl-*N*-{3-[(3-amino-2-hydroxypropanoyl)-amino]-2,2-dimethylpropyl}-5-(2,5-difluorophenyl) pentane amine | 5 |
| 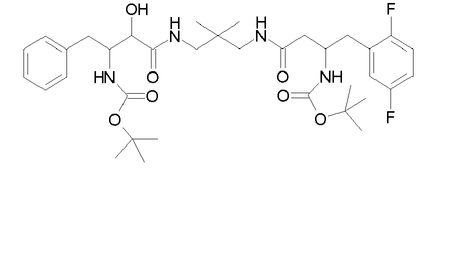 | 3-N-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 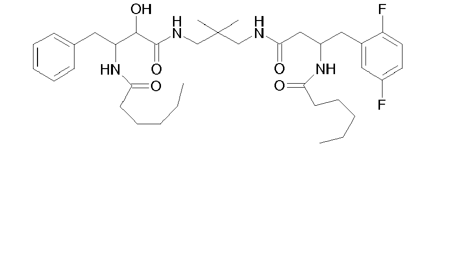 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 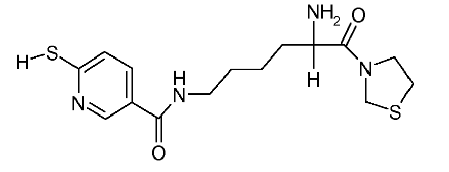 | *N*-[5-Amino-6-oxo-6-(1,3-th iazolidine-3-yl)hexyl]-6-mercapto nicotinamide | 8 |
| 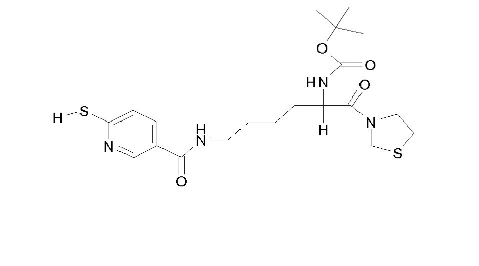 | 5-{[(6-Mercaptopyridine-3-yl)carbonyl]amino}-1-(1,3-thiazolidine-3-ylcarbonyl)pentyl carbamic acid (2,2 - dimethylethyl) ester | 9 |
| 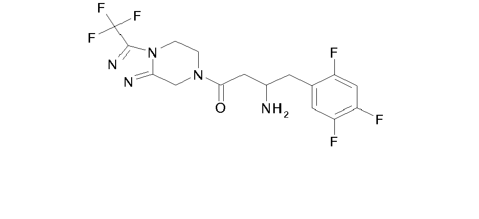 | 3-Oxo-1-(2,4,5-trifluorobenzyl)-3-[3-(trifluoromethyl)-5,6-dihydro-[1,2,4]triazolo[4,3-a]pyrazine-7(8*H*)-yl]propyl amine | 10 |
| 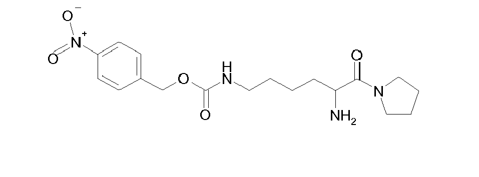 | 4-Nitrophenyl [5-amino-6-oxo-6-(1,3-pyrrolidine-3-yl) hexyl] carbamate | 11 |
| 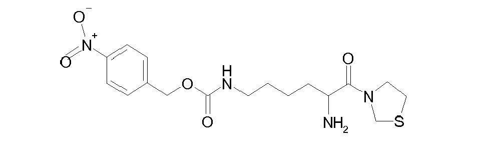 | 4-Nitrophenyl [5-amino-6-oxo-6-(1,3-thiazolidine-3-yl)hexyl] carbamate | 12 |
| 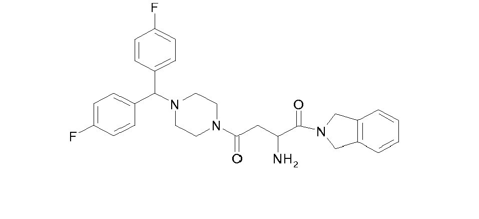 | 2-Amino-1-(1,3-dihydro-2*H*-isoindol-2-yl)-4-(4-bis(4-fluorophenyl)-methylpiperazine-1-yl) butane-1,4-dione | 13 |
| 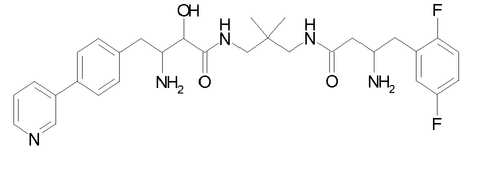 | 3-Amino-5-(4-(3-pyridinyl)-phenyl)-*N*-{3-[(3-amino-2-hydroxypropanoyl)-amino]-2,2-dimethylpropyl}-5-(2,5-difluorophenyl) pentane amide | 14 |
| 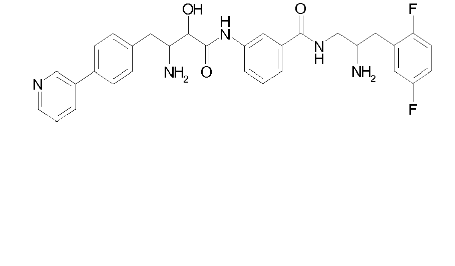 | 3-[(3-Amino-2-4-(4-(3-pyridinyl)- phenyl) hydroxybu-tanoyl)-amino]-*N*-(2-amino-5-(2,4-difluorophenyl) pentyl) ben-zamide | 15 |
| 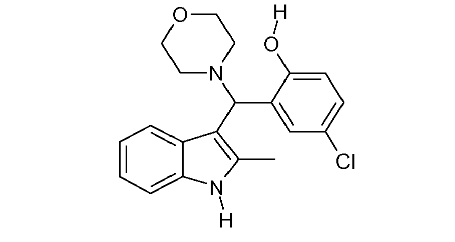 | 4-Chloro-2-[(2-methyl-1*H*-indol-3-yl)(morpholin-4-yl) methyl]- benzenolate | 16 |

In other words: A number of compounds (including known inhibitors of DPIV or of enzymes having DPIV-analogous enzymatic activity, particularly inhibitors of DPIV and/or of DP8 and/or of DP9), and other - novel - compounds could be successfully selected as steric inhibitors of DPIV or of enzymes having a DPIV-analogous enzymatic activity. *In-silico-* and *in-vitro* methods could be used for a determination whether said compound binds to said binding site within the central pore of DPIV or of the enzyme having DPIV-analogous enzymatic activity wherein the crystal structure of DPIV or of the enzyme having DPIV-analogous enzymatic activity is used as a target.

In preferred embodiments of the method according to the invention, said determination whether said compound binds as a ligand or steric inhibitor to said binding site within the central pore of DPIV or of the enzyme having DPIV-analogous enzymatic activity comprises at least one feature selected from the group consisting of
(i) a determination of the binding affinity (Ki value);
(ii) a determination of the distance between the active site (Glu205-Glu206) or enzymes having DPIV-analogous enzymatic activity and the central pore binding site by at least 10 Angström preferably by 20 Angström;
(iii) a determination of the hydrophobicity of the ligand or steric inhibitor, the molecular dimension of the ligand or steric inhibitor and degree of steric hindrance of access to the Glu205 - Glu206 active site by the ligand or steric inhibitor; and
(iv) an identification of at least 1 of 3 amino acid residues at said binding site within the central pore of DPIV or of the enzyme having DPIV-analogous enzymatic activity which are in close vicinity to the steric inhibitor or ligand.

As described in more detail in the experimental part of the description as a preferred embodiment, *in-silico* and *in-vitro* methods are suitable in accordance with this preferred embodiment of the invention for the determination.

The binding affinity (Ki value), the ligand hydrophobicity values and molecular dimension values of the ligand as well as the ligand's sterical hindrance can be determined from crystal structure data, and at least 1 of 3 amino acids of the central pore binding site may be identified by a skilled person by referring to known technical data sets derivable from well-known and best described in connection to crystal structure data.

Surprisingly, a more than 90 % correlation of the values determined on different routes could be obtained. This fact shows that the methods of the invention are highly valuable for an evaluation of the suitability of compounds as DPIV steric inhibitors.

In a further preferred and advantageous method of the invention, said data determined are correlated to the inhibitory effect of said chemical compounds on the DNA synthesis and proliferation of cells expressing DPIV or the enzyme having DPIV-analogous enzymatic activity, preferably the DNA synthesis and proliferation of lymphocytes, dermal cells, e. g. keratinocytes, fibroblasts and sebocytes and other cells expressing the enzyme.

It was surprisingly found by the inventors that a relatively close match of the data obtained from the in-silico analysis and the biologically obtained data could be achieved. This fact shows that both routes achieve reliable data sets allowing a theoretical evaluation which may be much more rapid - and nevertheless in accordance with the facts: Based on such data obtained by the methods of the invention, a prediction of properties may be made, and unwanted side effects of inhibitors may be avoided so that effects of DPIV inhibitors developed for treating Diabetes mellitus type II on immunologic functions (and vice versa) can be prevented.

The latter recognition resulted - quite surprisingly - in a novel use of the method according to the above detailed description for a characterization of pharmacokinetic, pharmacodynamic, toxicologic and immunotoxicologic effects of steric inhibitors and ligands of DPIV or of an enzyme having DPIV-analogous enzymatic activity, or as an alternative embodiment of the invention, into a novel use of the method according to the present invention for a development or an optimization of steric inhibitors of DPIV or of an enzyme having DPIV-analogous enzymatic activity.

In particularly preferred embodiments of the invention such uses allow the utilization of data based on an "in-silico" analysis for an evaluation and assessment of certain important properties of compounds suitable as steric inhibitors of DPIV or of enzymes having a DPIV-analogous enzymatic activity.

This may be desired, and hence particularly preferred in accordance with the invention, for the use of the method as described above in detail, for an evaluation and validation of ligands or steric inhibitors of DPIV or of an enzyme having DPIV-analogous enzymatic activity for their ability to be used in the medical field. In view of the pivotal importance of DPIV and of enzymes having DPIV-analogous enzymatic effect, a characterization, evaluation and validation of steric inhibitors of DPIV or of enzymes having DPIV-analogous enzymatic activity was needed and sought for.

In even more preferred uses of the invention, the purpose is an evaluation and validation of inhibitors or ligands of DPIV or of an enzyme having DPIV-analogous enzymatic activity for their ability to be used in the treatment, or in the manufacture of a medicament for the treatment, of Diabetes mellitus type II. In DPIV inhibitory processes connected to the treatment of diabetes, soluble DPIV is the main catalyst to which an access by means of both pores is available. Unwanted immunoregulatory side effects can be prevented by selecting suitable drug or steric inhibitor compounds. Due to the findings of the invention, suitable compounds - including medicaments showing no side effects - for achieving the object can easily be determined and validated.

In another particularly preferred embodiment of use of the present methods, an evaluation and validation of ligands or steric inhibitors of DPIV or of an enzyme having DPIV-analogous enzymatic activity for their ability to be used in the treatment, or in the manufacture of a medicament for the treatment, of inflammatory diseases. Preferably such a treatment of inflammatory diseases may have attempted a treatment of autoimmune diseases, allergies and transplant rejection. In the treatment of inflammatory diseases, particularly having an immune regulation etiology, membrane-bound cellular DPIV Is involved. As can be seen from Figure 1, due to the attachment of the DPIV molecule to the membrane, the side pore access to the inner area of the molecule my be obstructed, and an access to the active site of DPIV will easily be possible only via the central pore. Said pore entrance may be prevented from being passed by molecules acting as DPIV steric inhibitors or ligands by binding to the central pore binding site. Due to the findings of the invention, suitable compounds - including medicaments showing no side effects - for achieving the object can easily be determined and validated.

In a further preferred embodiment of the invention, the use is directed to an evaluation and validation of ligands or steric inhibitors of DPIV or of an enzyme having DPIV-analogous enzymatic activity for their ability to be used in the treatment, or in the manufacture of a medicament for the treatment, of chronic or acute neuronal diseases. Due to the findings of the invention, suitable compounds - including medicaments showing no side effects - for achieving the object can easily be determined and validated.

Finally, the invention relates to pharmaceutical preparations, which comprise at least one compound selected from the group consisting of the compounds of the following Table I:

**Table I**

| **Structure** | **IUPAC name** | **No.** |
|---|---|---|
| 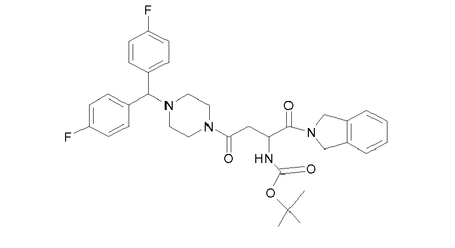 | 2-Oxy-methoxy-amino-1-(1,3-dihydro-2*H*-isoindol-2-yl)-4-(4-bis(4-fluorophenyl)methyl piperazine-1-yl)butane-1,4-dione | 3 |
| 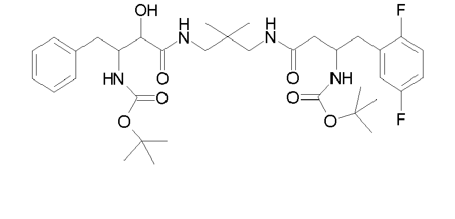 | 3-N-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 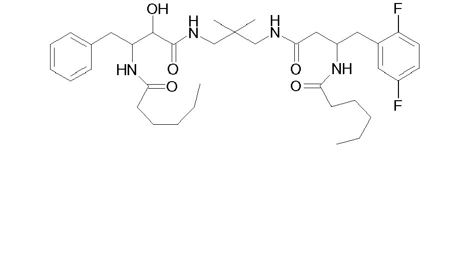 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 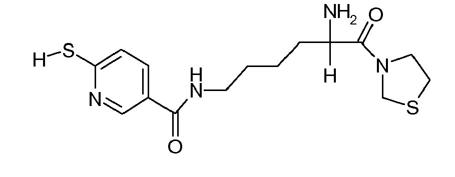 | *N*-[5-Amino-6-oxo-6-(1,3-thiazolidine-3-yl)hexyl]-6-mercapto nicotinamide | 8 |
| 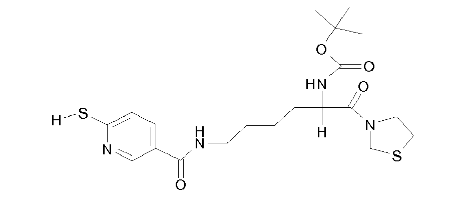 | 5-{[(6-Mercaptopyridine-3-yl)carbonyl]amino}-1-(1,3-thiazolidine-3-yl carbonyl)pentyl carbamic acid (2, 2 - dimethylethyl) ester | 9 |
| 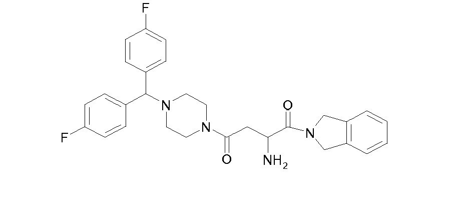 | 2-Amino-1-(1,3-dihydro-2*H*-isoindol-2-yl)-4-(4-bis(4-fluorophenyl)-methylpiperazine-1-yl) butane-1,4-dione | 13 |
| 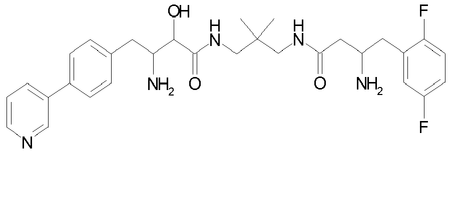 | 3-Amino-5-(4-(3-pyridinyl)-phenyl)-*N*-{3-[(3-amino-2-hydroxypropanoyl)-amino]-2,2-dimethylpropyl}-5-(2,5-difluorophenyl) pentane amide | 14 |
| 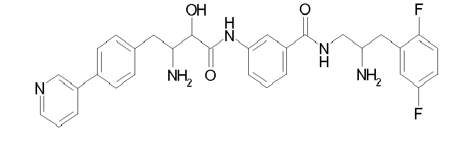 | 3-[(3-Amino-2-4-(4-(3-pyridinyl)- phenyl) hydroxybutanoyl)-amino]-*N*-(2-amino-5-(2,4-difluorophenyl) pentyl) benzamide | 15 |

The above pharmaceutical compositions comprise at least one, preferably contain one, of the above novel compounds of Table I, optionally in combination with per se known pharmaceutically acceptable auxiliary substances or excipients. As could be found by the present invention, these pharmaceutical preparations may be used with advantage in the manufacture of medicaments for the treatment of inflammatory diseases, preferably autoimmune diseases, allergies, transplant rejection, chronic inflammatory skin diseases as well as chronic or acute neuronal diseases.

Consequently, the invention furthermore relates of the compounds selected from the group consisting of the compounds having the following structures in the medical field:

**Table I**

| **Structure** | **IUPAC name** | **No**. |
|---|---|---|
| 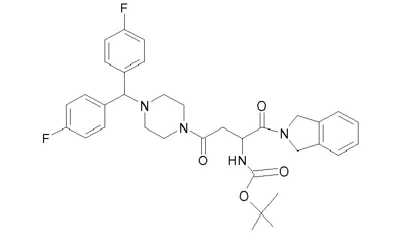 | 2-Oxy-methoxy-amino-1-(1,3-dihydro-2*H*-isoindol-2-yl)-4-(4-bis(4-fluorophenyl)methyl pipe-razine-1-yl)butane-1,4-dione | 3 |
| 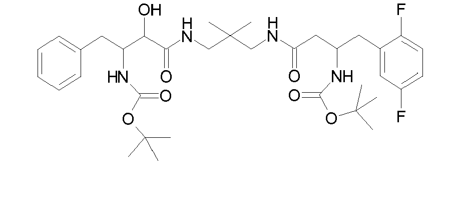 | 3-*N*-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 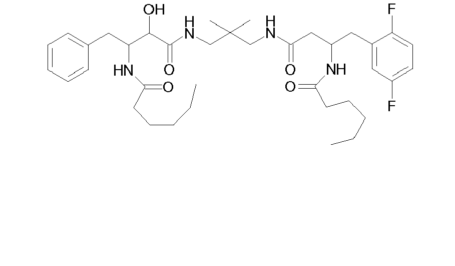 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 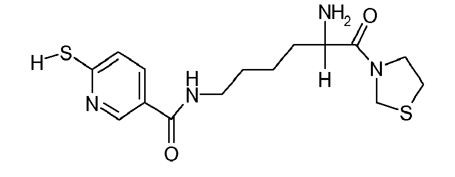 | *N*-[5-Amino-6-oxo-6-(1,3-thiazolidine-3-yl)hexyl]-6-mercapto nicotinamide | 8 |
| 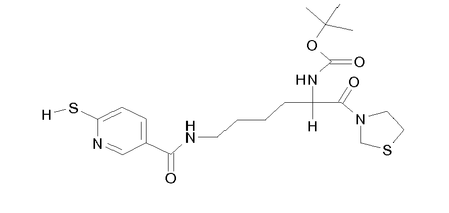 | 5-{[(6-Mercaptopyridine-3-yl)carbonyl]amino}-1-(1,3-thiazolidine-3-yl carbonyl)pentyl carbamic acid (2, 2 - di-methylethyl) ester | 9 |
| 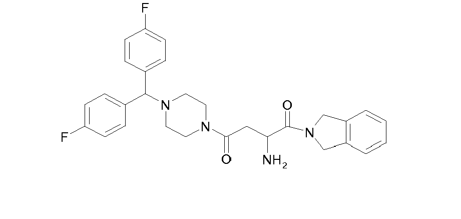 | 2-Amino-1-(1,3-dihydro-2*H*-isoindol-2-yl)-4-(4-bis(4-fluorophenyl)-methylpiperazine-1-yl) butane-1,4-dione | 13 |
| 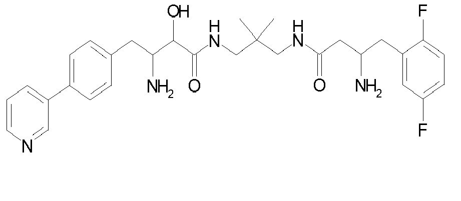 | 3-Amino-5-(4-(3-pyridinyl)-phenyl)-*N*-{3-[(3-amino-2-hydroxypropanoyl)-amino]-2,2-dimethylpropyl}-5-(2,5-difluorophenyl) pentane amide | 14 |
| 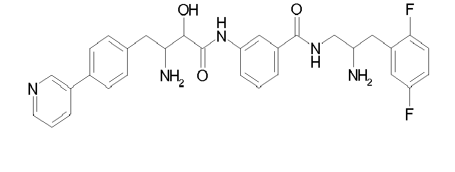 | 3-[(3-Amino-2-4-(4-(3-pyridinyl)- phenyl) hydroxybu-tanoyl)-amino]-*N*-(2-amino-5-(2,4-difluorophenyl) pentyl) benzamide | 15 |

Specifically, the invention relates to the use of the compounds of the aforementioned Table I as steric inhibitors for dipeptidyl peptidase IV (DPIV) or for enzymes having DPIV-analogous enzymatic activity *in silico* or *in vitro.* Hence, these compounds may be used advantageously for the characterization of pharmacokinetic, pharmacodynamic, toxicologic and immunotoxicologic effects of inhibitors or ligands of DPIV or of an enzyme having DPIV-analogous enzymatic activity or for a development or an optimization of inhibitors or ligands of DPIV or of an enzyme having DPIV-analogous enzymatic activity. Hence, an evaluation and validation of inhibitors or ligands of DPIV or of an enzyme having DPIV-analogous enzymatic activity for their ability to be used in the treatment, or in the manufacture of a medicament for the treatment of Diabetes mellitus type II, for the treatment of inflammatory diseases, preferably of autoimmune diseases, allergies, transplant rejections, chronic inflammatory skin diseases, or for the treatment of chronic or acute neuronal diseases may advantageously be achieved.

Furthermore, and as described above - and also shown in the experimental part of the description in more detail, the compounds of Table I were found by the inventors to indirectly or sterically inhibit DPIV or enzymes having a DPIV-analogous enzymatic activity by binding to the DPIV His363 - Glu 361 - Glu 408 binding site within the central pore.

Finally, the above novel compounds shown in Table I may be used for the manufacture of for manufacturing a medicament for the treatment of
- inflammatory diseases, preferably of autoimmune diseases, allergies, transplant rejection;
- chronic inflammatory diseases; or
- chronic or acute neuronal diseases.

The invention was described by referring to the preferred embodiments thereof, which are given for explanatory purposes for supporting a better understanding of the invention, but this should not be construed to limit the invention to these preferred embodiments, only.

### Methods

### Measurement of the enzymatic activity of DPIV and related enzymes

The inhibition of the enzymatic activity of DPIV was measured by using purified, recombinant human DPIV (final enzyme concentration approx. 1 nM). The assay was performed in 0.05 M TRIS/HCl buffer, pH 7.5, supplemented with 0.05 % Triton^{®} (v/v), 0.05 % BSA (w/v), 2 mM MgCl₂.

The enzymatic activity of DPIV was assessed by the hydrolysis of the fluorogenic substrate Bis-(L-Alanyl-Prolyl)-Rhodamin-110 (Bis-Trifluoroacetate; abbreviation: (Ala-Pro)₂-R110). The final substrate concentration was 0.5 µM.

The assay was performed in white microtitre plates for fluorescence measurements. The test items, substrate and enzyme, were diluted in assay buffer. The highest test item concentrations used were 25 µM. For the calculation of IC₅₀ values, at least 16 log2 dilutions of each test item were analyzed. As controls, the DPIV activity in the absence of test items as well as the spontaneous hydrolysis of the substrate was determined.

The release of the fluorescent hydrolysis product Rhodamine 110 was measured at an excitation wavelength of 485 nm and an emission wavelength of 530 nm by using a microtitre fluorescence reader immediately after substrate addition as well as after 30, 60 and 120 min.

### Inhibition of proliferation of human peripheral blood mononuclear cells and T lymphocytes

Peripheral blood mononuclear cells (PBMC) from healthy human volunteers were freshly isolated by density gradient centrifugation. T lymphocytes (T cells, Tc) were isolated from PBMC fractions via nylon wool adherence. Cells were cultured in standard cell culture media and stimulated by addition of 1 µg/ml Phytohemagglutinine for 48 hours in 96 well flat-bottom microtitre plates. Test compounds were added at concentration ranges from 0.1 to 250 µM over the total assay period.

DNA synthesis of proliferating PBMC was assessed by incorporation of nucleotide analogue bromdesoxyuridine (BrdU) and subsequent detection of optical density (OD) at 450 nm.

DNA synthesis of proliferating T cells was determined by incorporation of radio labelled tritium thymidine and subsequent radio detection.

Data output was based on raw data and calculation of relative proliferation response in relation to PHA-activated T cells in the absence of test compound (100 % control).

The IC₅₀ value of proliferation suppression was assessed by graphical evaluation.

### Computational details

All enzymatic data are related to the DPIV crystal structure, solved by Rasmussen et al [H. B.Rasmussen et al. Nat. Struct. Biol., 2003, 10, 19 - 25].

The in detail study of the protein surface around the active site pocket revealed a shallow dell within the central pore (tunnel) between the active site pocket and the central access. Several docking runs with known DPIV inhibitors, that include the region around both, the active site pocket and the shallow dell discussed here, resulted in binding constants for the shallow dell region within one or two orders of magnitude of those obtained for the binding of the respective inhibitor within the active site pocket. Restriction of the region considered in the docking procedures and application on a wide range of compounds proved that this shallow dell is a possible alternative binding site; this binding site is named central pore binding site.

For geometric studies, the active site pocket is represented by Glu205, Glu206, and Arg358 and the central pore binding site is represented by Glu361, His363 and Glu408.

Two critical distances are included in this model. The first distance for characterizing the position of small molecules within the central pore binding site towards the active site pocket is that between the carbonyl carbon of Glu206 and the centre of mass of the steric inhibitor/ligand (rₐₛ). The second one is that between the centre access point of the DPIV central pore and the centre of mass of the steric inhibitor/ligand (r_{ac}), that characterizes the position of the steric inhibitor/ligand towards the entrance of the central pore. The *van der Waals* surface A_{vdw} of the steric inhibitor/ligand is used as a variable that relates to the special obstruction of the DPIV central access pore. Furthermore, short polar contacts between the steric inhibitors/ligands and at least two of the residues that represent the central pore binding site are crucial for stable thermodynamic interactions.

### Molecular docking and quantum chemical calculations

The docking studies were carried out with the Autodock 4 package [www.Scripps.edu] on a 4 CPU computer system under Windows 7.

For the docking procedures, we used the A-Monomer of the dimeric DPIV crystal structure. The water molecules within the region of the monomer considered here were removed before. For all Dockings, 256 Generic Algorithm runs were carried out.

logP values were calculated with the method implemented as optional add-on in the ChemSketch software package [www.acdlabs.com].

Molecular volumes and surfaces were obtained by using the COSMO method [A. Klamt et al. Journal of the Chemical Society, Perkin Transaction 2, 799 (1993)], that is implemented in *MOPAC2002* [http://www.cache.fujitsu.com/mopac/index.shtml].

### Multi linear Regression Analysis model of T cell proliferation inhibition

### Regression model:

The IC₅₀ of the suppression of DNA synthesis of T cells (DNA-Supp.) is given in µM and appears in the regression equation as natural logarithm. The most expedient distance related parameter for the model discussed here is the difference of the squares of the two distances considered here, (rₐₛ² - r_{ac}²). Their representation in squared shape seems necessary to avoid the occurrence of equal differences for different positions, as it would be probable for simple differences of distances.

T cell proliferation inhibition (DNA-Supp.) is calculated in dependence on three variables, the natural logarithm of the binding constant of the ligand in the DPIV central pore binding site (K_{cp}), the distance parameter (rₐₛ² - r_{ac}²) and the number of free amino groups (N_{A}) of the ligand. R = 9.955. The smaller the binding constant of the ligand in the DP IV central pore binding site (K_{cp}), the better the binding.

The results are shown in Table III. As it is obvious from Table III, the correlation between the measured T cell proliferation inhibition (measured DNA-Supp.) and the calculated T cell proliferation inhibition (calculated DNA Supp.) is more than 90 %.

The training data set contains an overall number of 16 structurally diverse chemical compounds with DNA-Supp. values that range from 3.42 to 200.

**Table III: Comparison of different DPIV inhibitors with respect to their enzymatic DPIV and DP8/9 inhibition, the suppression of DNA synthesis and their binding characteristics at the central pore binding site.**

| Structure | IC₅₀(Tc) (µM) Measured DNA-Supp. | IC₅₀(Tc) (µM) Calculated DNA-Supp. DPIV structure based | IC₅₀(DPIV) (µM) | IC₅₀(DP8/9) (µM) | rₐₛ (Å) | r_{ac} (Å) | K_{cp} (µM) | NA |
|---|---|---|---|---|---|---|---|---|
| 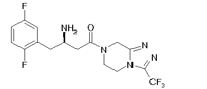 | 200 | 146 | 0.072 | 30.6 | 14 | 22 | 0.4 | 1 |
| 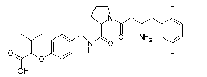 | 81 | 45 | 0.480 | > 25.00 | 15 | 18 | 0.04 | 1 |
| 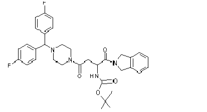 | 4 | 5 | > 25.00 | > 25.00 | 19 | 20 | 0.005 | 0 |
| 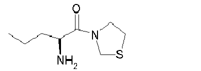 | 113 | 181 | 0.26 | 1.73 | 17 | 16 | 6.75 | 1 |
| 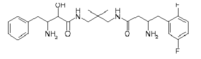 | 200 | 134 | 0.08 | 1.4 | 14 | 20 | 0.011 | 2 |
| 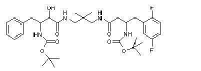 | 8 | 10 | > 25.00 | > 25.00 | 24 | 10 | 1.79 | 0 |
| 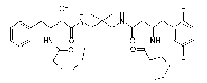 | 10 | 16 | > 25.00 | > 25.00 | 20 | 13 | 0.929 | 0 |
| 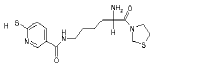 | 90 | 70 | 0.09 | 0.001 | 22 | 14 | 2.76 | 1 |
| 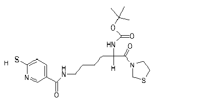 | 17 | 19 | > 25.00 | > 25.00 | 17 | 20 | 0.147 | 0 |
| 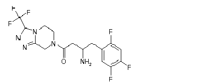 | 148 | 113 | 0.001 | 15.3 | 13 | 21 | 0.204 | 1 |
| 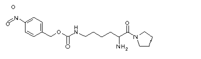 | 22 | 67 | 0.15 | 0.022 | 18 | 16 | 0.453 | 1 |
| 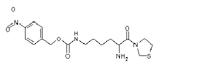 | 26 | 55 | 0.05 | 0.002 | 20 | 16 | 0.421 | 1 |
| 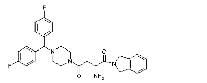 | 6 | 10 | 8.9 | 0.004 | 20 | 15 | 0.003 | 1 |
| 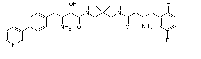 | 65 | 130 | 0.096 | 0.81 | 18 | 18 | 0.035 | 2 |
| 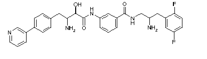 | 24 | 25 | 0.775 | 8.5 | 18 | 14 | 0.0009 | 2 |
| 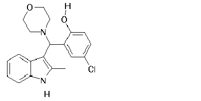 | 3 | 2 | > 25.00 | > 25.00 | 31 | 4 | 0.282 | 0 |

### Legend:

Dataset used for calculation: In(K_{cp}), rₐₛ² - r_{ac}², N_{A}: For details of the model please refer to the subparagraph 'Multi linear Regression Analysis model of T cell proliferation inhibition' within the 'chapter 'Computational details'; DNA-Supp.: suppression of DNA synthesis of T cells;
rₐₛ: distance between the carbonyl carbon atom of GLU206 and the centre of mass of the ligand;
r_{ac}: distance between the centre of the central access pore and the centre of mass of the ligand;
K_{cp}: Binding constant of the ligand in the central pore binding site (calculated in the docking procedure);
N_{A}: Number of free amino groups > 25: No inhibition

### Regression model data:

Number of calibration compounds: 16
Number of variables: 3
Coefficient of determination (r²): 0.913
Standard deviation (SD): 0.467

## Claims

1. A composition comprising (i) at least one member selected from dipeptidyl peptidase IV (DPIV) and an enzyme having DPIV-analogous enzymatic activity; and (ii) at least one steric inhibitor selected from steric inhibitors of dipeptidyl peptidase IV (DPIV) and of an enzyme having DPIV-analogous enzymatic activity, wherein said at least one steric inhibitor is bound to a binding site within the central pore of DPIV or of the enzyme having DPIV-analogous enzymatic activity.

2. The composition according to claim 1, wherein said at least one steric inhibitor is bound to a binding site within the central pore of DPIV or of the enzyme having DPIV-analogous enzymatic activity, which is distant from the active site of DPIV (Glu205 - Glu206) or of the enzyme having DPIV-analogous enzymatic activity by at least 10 Angström (1 nm), preferably by 20 Angström (2 nm), preferably wherein said at least one steric inhibitor is bound to at least one of the amino acids His363 - Glu361 - Glu408 within the binding site of the central pore of DPIV.

3. The composition according to claim 1 or claim 2, wherein the at least one steric inhibitor is selected from the group consisting of the following compounds:
| **Structure** | **IUPAC name** | **No.** |
|---|---|---|
| 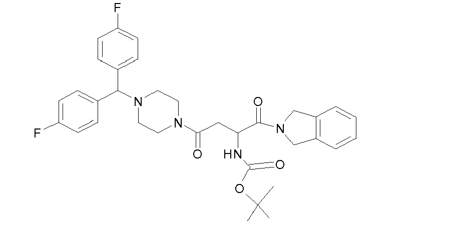 | 2-Oxy-methoxy-amino-1-(1,3-dihydro-2H-isoindol-2-yl)-4-(4-bis(4-fluorophenyl)methyl piperazine-1-yl)butane-1,4-dione | 3 |
| 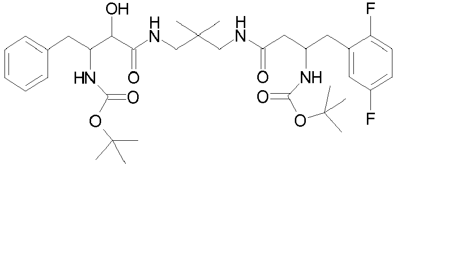 | 3-N-tert.-Butylcarbamato-5-phenyl-N-{3-[(3-amino-2-hydroxypropanoyl)-amino]-2,2-dimethylpropyl}-5-(2,5-difluorophenyl) pentane 3-amine | 6 |
| 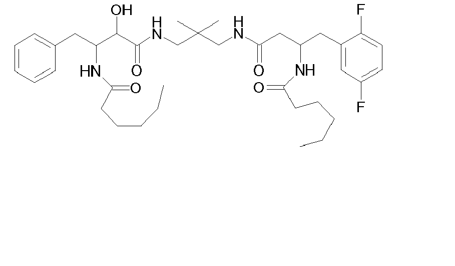 | 3-Amino-5-phenyl-N-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 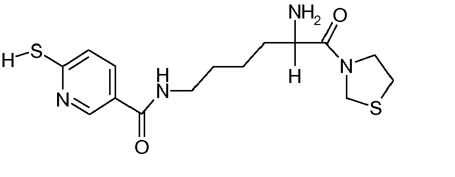 | N-[5-Amino-6-oxo-6-(1,3- thiazolidine-3-yl)hexyl]-6-mercapto nicotinamide | 8 |
| 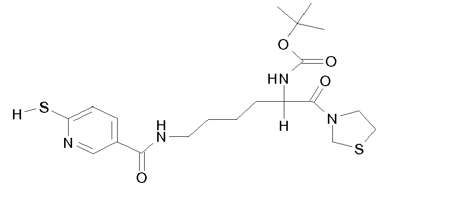 | 5-{[(6-Mercaptopyridine-3-yl)carbonyl]amino}-1-(1,3-thiazolidine-3-ylcarbonyl)pentyl carbamic acid (2, 2 - dimethylethyl) ester | 9 |
| 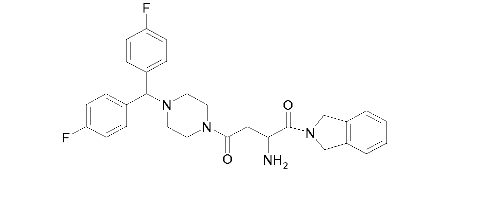 | 2-Amino-1-(1,3-dihydro-2H-isoindol-2-yl)-4-(4-bis(4-fluorophenyl)-methylpiperazine-1-yl) butane-1,4-dione | 13 |
| 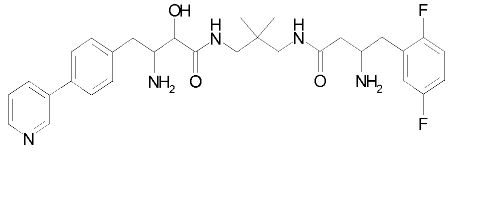 | 3-Amino-5-(4-(3-pyridinyl)-phenyl)-N-{3-[(3-amino-2-hydroxypropanoyl)-amino]-2,2-dimethylpropyl}-5-(2,5-difluorophenyl) pentane amide | 14 |
| 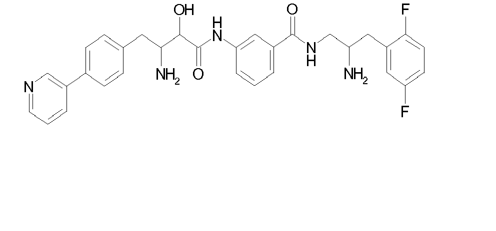 | 3-[(3-Amino-2-4-(4-(3-pyridinyl)- phenyl) hydroxybu-tanoyl)-amino]-*N*-(2-amino-5-(2,4-difluorophenyl) pentyl) benzamide | 15 |

4. A method for an *in silico* or *in vitro* characterization of chemical compounds as steric inhibitors for dipeptidyl peptidase IV (DPIV) or for enzymes having DPIV-analogous enzymatic activity, which method comprises the steps of
(i) selecting a compound for the characterization;
(ii) bringing said selected compound into contact with dipeptidyl peptidase IV (DPIV) or with an enzyme having DPIV-analogous enzymatic activity;
(iii) binding said compound to a binding site within the central pore, preferably to at least one of the amino acids His363 - Glu361 - Glu408, which is distant from the active site of DPIV (Glu205 - Glu206) or of the enzyme having DPIV-analogous enzymatic activity by at least 10 Angström (1 nm), preferably by 20 Angström (2 nm);
(iv) determining whether said compound binds to said binding site within the central pore of DPIV or of the enzyme having DPIV-analogous enzymatic activity and restrict or even prevent the access to the active site;
wherein the crystal structure of DPIV or models of enzymes having DPIV-analogous enzymatic activity is used as a target.

5. The method according to claim 4, calibrated by using the following compounds as inhibitors or ligands for dipeptidyl peptidase IV (DPIV) or for enzymes having DPIV-analogous enzymatic activity:
| **Structure** | **IUPAC name** | **No.** |
|---|---|---|
| 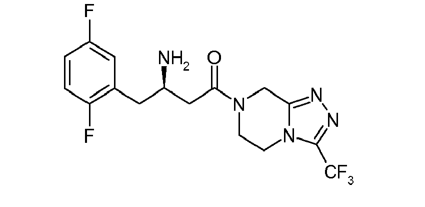 | (1*R*)-1-(2,5-Difluorobenzyl)-3-oxo-3-[3-(trifl uoromethyl)-5,6-dihydro[1,2,4]triazolo[4,3-a]pyrazine-7(8H)-yl]propylamine | 1 |
| 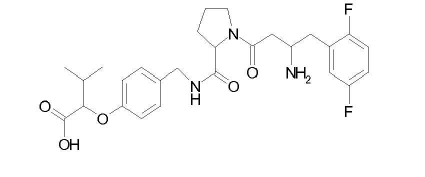 | 2-[({[1-(3-Amino, 2-(2, 5)-difluorophenylbutanoyl)pyrrolidin-2-yl]carbonyl)amino)-p-phenoxy]-3-methyl butanoic acid | 2 |
| 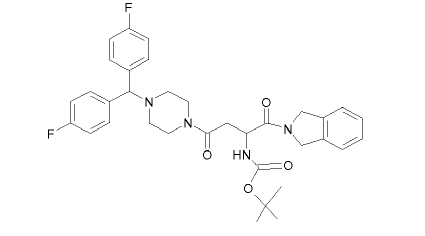 | 2-Oxy-methoxy-amino-1-(1,3-dihydro-2H-isoindol-2-yl)-4-(4-bis(4-fluorophenyl)methyl piperazine-1-yl)butane-1,4-dione | 3 |
| 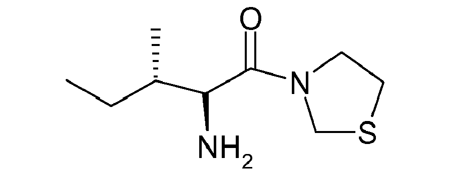 | (1*S*,2*S*)-2-Methyl-1-(1,3-thiazolidine-3-ylcarbonyl) butyl amine | 4 |
| 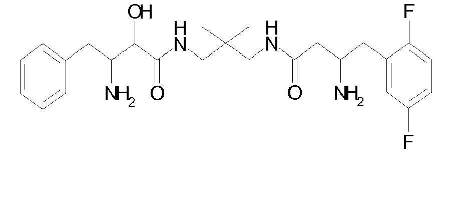 | 3-Amino-5-phenyl-*N*-{3-[(3-amino-2-hydroxypropanoyl)-amino]-2,2-dimethylpropyl}-5-(2,5-difluorophenyl) pentane amine | 5 |
| 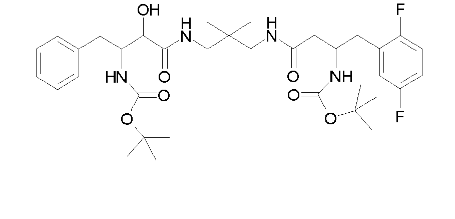 | 3-N-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 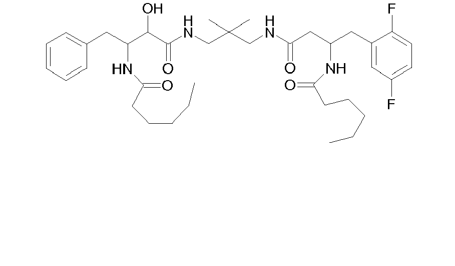 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 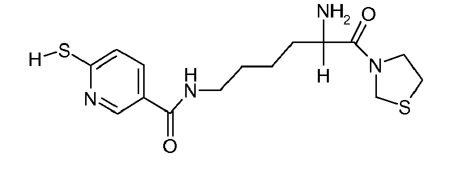 | *N*-[5-Amino-6-oxo-6-(1,3-thiazolidine-3-yl)hexyl]-6-mercapto nicotinamide | 8 |
| 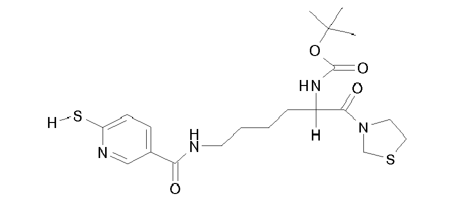 | 5-{[(6-Mercaptopyridine-3-yl)carbonyl]amino}-1-(1,3-thiazolidine-3-ylcarbonyl)pentyl carbamic acid (2, 2 - dimethylethyl) ester | 9 |
| 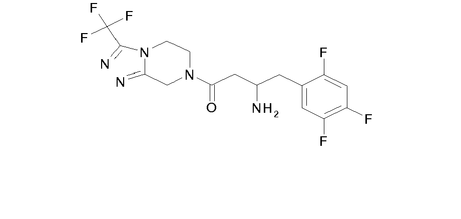 | 3-Oxo-1-(2,4,5-trifluorobenzyl)-3-[3-(trifluoromethyl)-5,6-dihydro-[1,2,4]triazolo[4,3-a]pyrazine-7(8*H*)-yl]propyl amine | 10 |
| 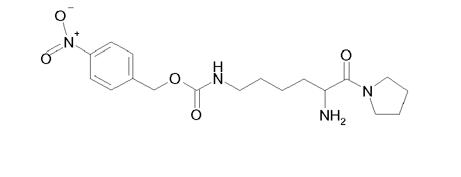 | 4-Nitrophenyl [5-amino-6-oxo-6-(1,3-pyrrolidine-3-yl) hexyl] carbamate | 11 |
| 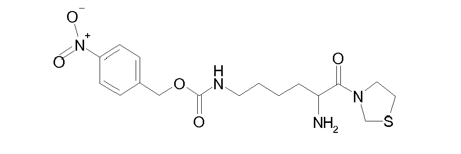 | 4-Nitrophenyl [5-amino-6-oxo-6-(1,3-thiazolidine-3-yl)hexyl] carbamate | 12 |
| 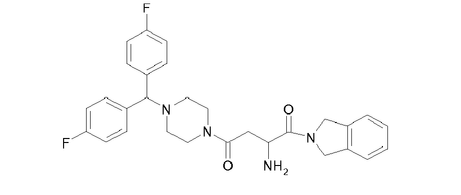 | 2-Amino-1-(1,3-dihydro-2*H*-isoindol-2-yl)-4-(4-bis(4-fluorophenyl)-methylpiperazine-1-yl) butane-1,4-dione | 13 |
| 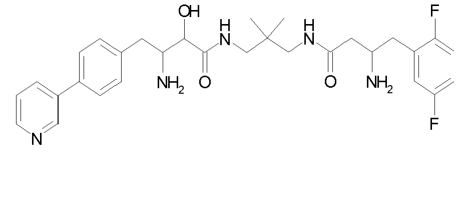 | 3-Amino-5-(4-(3-pyridinyl)-phenyl)-*N*-{3-[(3-amino-2-hydroxypropanoyl)-amino]-2,2-dimethylpropyl}-5-(2,5-difluorophenyl) pentane amide | 14 |
| 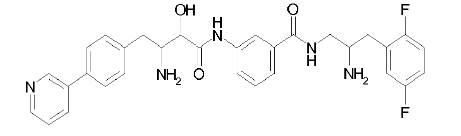 | 3-[(3-Amino-2-4-(4-(3-pyridinyl)-phenyl) hydroxybutanoyl)-amino]-*N*-(2-amino-5-(2,4-difluorophenyl) pentyl) benzamide | 15 |
| 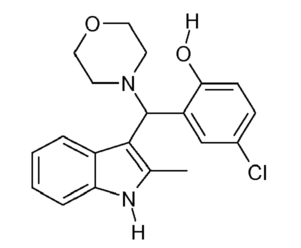 | 4-Chloro-2-[(2-methyl-1*H*-indol-3-yl)(morpholin-4-yl) methyl]-benzenolate | 16 |

6. The method according to claim 4 or claim 5, wherein said determination whether said compound binds as a ligand or steric inhibitor to said binding site within the central pore of DPIV or of the enzyme having DPIV-analogous enzymatic activity comprises at least one feature selected from the group consisting of
(i) a determination of the binding affinity (Ki value);
(ii) a determination of the distance between the active site (Glu205-Glu206) or enzymes having DPIV-analogous enzymatic activity and the central pore binding site by at least 10 Angström preferably by 20 Angström;
(iii) a determination of correlatives of the hydrophobicity of the ligand or steric inhibitor, the molecular dimension of the ligand or steric inhibitor and degree of steric hindrance of access to the Glu205 - Glu206 active site by the ligand or steric inhibitor; and
(iv) an identification of at least 1 of 3 amino acid residues at said binding site within the central pore of DPIV or of the enzyme having DPIV-analogous enzymatic activity which are in close vicinity to the ligand or steric inhibitor.

7. The method according to any of the claims 4 to 6, wherein said data determined are correlated to the inhibitory effect of said chemical compounds on the DNA synthesis and proliferation of cells expressing DPIV or the enzyme having DPIV-analogous enzymatic activity, preferably the DNA synthesis and proliferation of lymphocytes, dermal cells, e. g. keratinocytes, fibroblasts and sebocytes and other cells expressing the enzyme.

8. A use of the method according to any of the claims 4 to 7 for a characterization of pharmacokinetic, pharmacodynamic, toxicologic and immunotoxicologic effects of inhibitors or ligands of DPIV or of an enzyme having DPIV-analogous enzymatic activity, or for a development or an optimization of inhibitors or ligands of DPIV or of an enzyme having DPIV-analogous enzymatic activity.

9. The use of the method according to any of the claims 4 to 7 for an evaluation and validation of inhibitors or ligands of DPIV or of an enzyme having DPIV-analogous enzymatic activity for their ability to be used in the medical field.

10. The use of claim 9 for an evaluation and validation of inhibitors or ligands of DPIV or of an enzyme having DPIV-analogous enzymatic activity for their ability to be used in the treatment, or in the manufacture of a medicament for the treatment, of Diabetes mellitus type II, of inflammatory diseases, preferably of autoimmune diseases, allergies, transplant rejection, chronic inflammatory skin diseases or of chronic or acute neuronal diseases.

11. Pharmaceutical preparations, comprising at least one compound selected from the group consisting of:
| **Structure** | **IUPAC name** | **No.** |
|---|---|---|
| 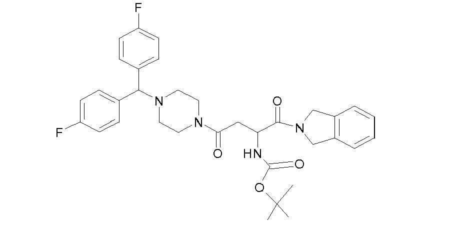 | 2-Oxy-methoxy-amino-1-(1,3-dihydro-2*H*-isoindol-2-yl)-4-(4-bis(4-fluorophenyl)methyl piperazine-1-yl)butane-1,4-dione | 3 |
| 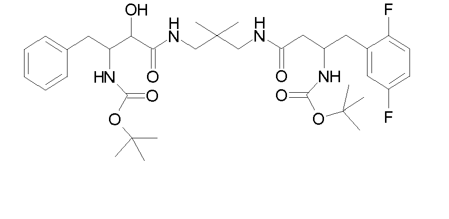 | 3-N-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 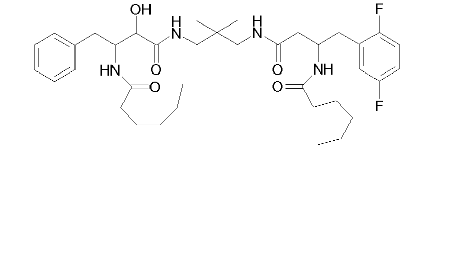 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 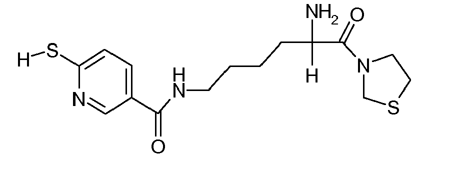 | *N*-[5-Amino-6-oxo-6-(1,3-thiazolidine-3-yl)hexyl]-6-mercapto nicotinamide | 8 |
| 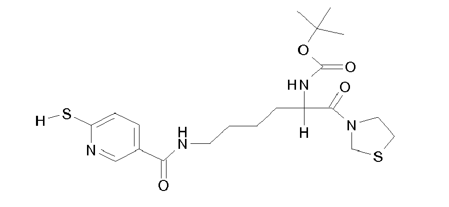 | 5-{[(6-Mercaptopyridine-3-yl)carbonyl]amino}-1-(1,3-thiazolidine-3-ylcarbonyl)pentyl carbamic acid (2, 2 - dimethylethyl) ester | 9 |
| 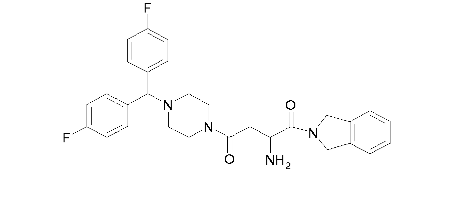 | 2-Amino-1-(1,3-dihydro-2*H*-isoindol-2-yl)-4-(4-bis(4-fluorophenyl)-methylpiperazine-1-yl) butane-1,4-dione | 13 |
| 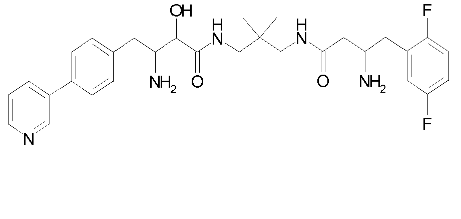 | 3-Amino-5-(4-(3-pyridinyl)-phenyl)-*N*-{3-[(3-amino-2-hydroxypropanoyl)-amino]-2,2-dimethylpropyl}-5-(2,5-difluorophenyl) pentane amide | 14 |
| 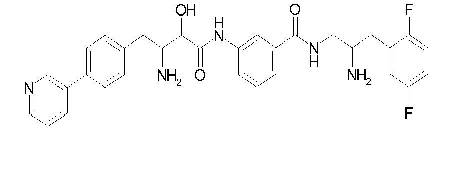 | 3-[(3-Amino-2-4-(4-(3-pyridinyl)- phenyl) hydroxybu-tanoyl)-amino]-*N*-(2-amino-5-(2,4-difluorophenyl) pentyl) benzamide | 15 |

12. A use of the compounds selected from the group consisting of the compounds having the following structures in the medical field:
| **Structure** | **IUPAC name** | **No.** |
|---|---|---|
| 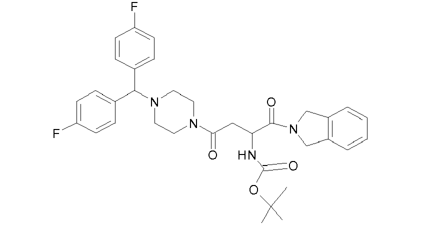 | 2-Oxy-methoxy-amino-1-(1,3-dihydro-2*H*-isoindol-2-yl)-4-(4-bis(4-fluorophenyl)methyl piperazine-1-yl)butane-1,4-dione | 3 |
| 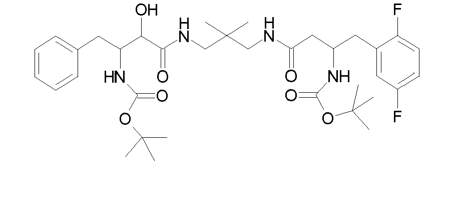 | 3-N-tert.-Butylcarbamato-5-phenyl-*N*-{3-[(3-amino-2-hydroxypropanoyl)-amino]-2,2-dimethylpropyl}-5-(2,5-difluorophenyl) pentane 3-amine | 6 |
| 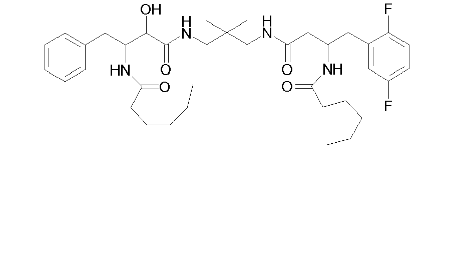 | 3-Amino-5-phenyl-*N*-{3-[(3-amino-1-oxohexyl-2-hydroxypropanoyl)amino-1-oxohexyl]-2,2-dimethylpropyl}-5-(2,5-difluorophenyl)pentane 3-N-tert.butyl carbamate | 7 |
| 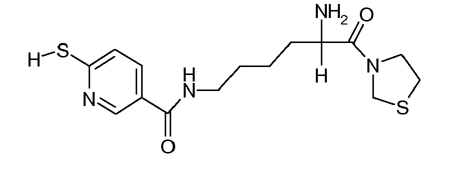 | *N*-[5-Amino-6-oxo-6-(1,3-thiazolidine-3-yl)hexyl]-6-mercapto nicotinamide | 8 |
| 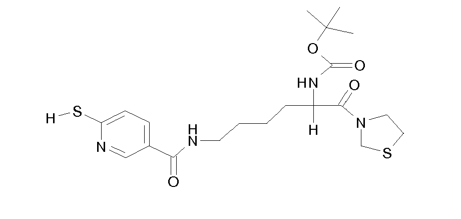 | 5-{[(6-Mercaptopyridine-3-yl)carbonyl]amino}-1-(1,3-thiazolidine-3-ylcarbonyl)pentyl carbamic acid (2, 2 - dimethylethyl) ester | 9 |
| 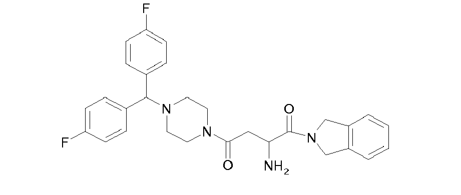 | 2-Amino-1-(1,3-dihydro-2*H*-isoindol-2-yl)-4-(4-bis(4-fluorophenyl)-methylpiperazine-1-yl) butane-1,4-dione | 13 |
| 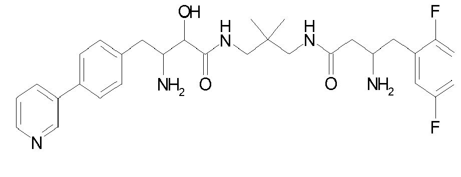 | 3-Amino-5-(4-(3-pyridinyl)-phenyl)-*N*-{3-[(3-amino-2-hydroxypropanoyl)-amino]-2,2-dimethylpropyl}-5-(2,5-difluorophenyl) pentane amide | 14 |
| 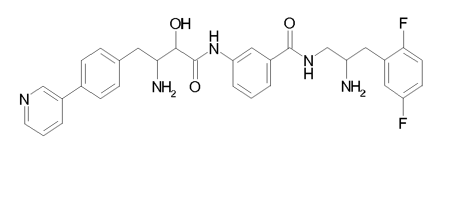 | 3-[(3-Amino-2-4-(4-(3-pyridinyl)-phenyl) hydroxybutanoyl)-amino]-*N*-(2-amino-5-(2,4-difluorophenyl) pentyl) benzamide | 15 |

13. The use of the compounds according to claim 12 as steric inhibitors for dipeptidyl peptidase IV (DPIV) or for enzymes having DPIV-analogous enzymatic activity *in silico* or *in vitro.*

14. A use of the compounds according to claim 12 for manufacturing a medicament for the treatment of
- inflammatory diseases, preferably of autoimmune diseases, allergies, transplant rejection;
- chronic inflammatory skin diseases; or
- chronic or acute neuronal diseases.
